# EUROPÄISCHE PATENTSCHRIFT

(11) **EP 2 908 836 B1**
(45) Veröffentlichungstag und Bekanntmachung des Hinweises auf die Patenterteilung: **11.09.2019**
(21) Anmeldenummer: 13740296.2
(22) Anmeldetag: 24.07.2013
(51) Int. Cl.: A61K 36/9068, A61P 35/00

(54) **PHARMAZEUTISCHE FORMULIERUNG ENTHALTEND CURCUMIN**
PHARMACEUTICAL FORMULATION CONTAINING CURCUMA
FORMULATION PHARMACEUTIQUE CONTENANT DU CURCUMA

(30) Priorität: 22.10.2012 DE 102012219219
(43) Veröffentlichungstag der Anmeldung: 26.08.2015
(73) Patentinhaber: BRIU GmbH, 61462 Königstein (DE)
(72) Erfinder: ROSE, Uwe-Bernd, 61462 Königstein (DE)
(74) Vertreter: Patent- und Rechtsanwälte Ullrich & Naumann
(86) Internationale Anmeldenummer: PCT/EP2013/065661
(87) Internationale Veröffentlichungsnummer: WO 2014/063844

(56) Entgegenhaltungen:
- WO-A1-2011/101859
- WO-A2-2009/061152
- ZHAO YAN-GUO ET AL: "Preparation of a bis-demethoxy curcumin microemulsion based on pseudo-ternary phase diagrams and an orthogonal test analysis", JOURNAL OF PESTICIDE SCIENCE,, Bd. 36, Nr. 2, 1. Januar 2011 (2011-01-01) , Seiten 248-251, XP009173239,
- WU XUEMEI ET AL: "Self-microemulsifying drug delivery system improves curcumin dissolution and bioavailability.", DRUG DEVELOPMENT AND INDUSTRIAL PHARMACY JAN 2011, Bd. 37, Nr. 1, Januar 2011 (2011-01), Seiten 15-23, XP002714380, ISSN: 1520-5762

## Beschreibung

Die Erfindung betrifft eine pharmazeutische Formulierung, die als Wirkstoff Curcumin oder ein Curcuminderivat enthält.

Curcumin lässt sich aus natürlichen Quellen (beispielsweise javanischer Gelbwurz) extrahieren oder synthetisch herstellen. Curcumin weist antineoplastische Eigenschaften auf, jedoch lassen sich keine für eine systemische Wirkung erforderlichen Blutspiegel herstellen, da Curcumin wasserunlöslich ist.

WO 2011/101859 A1 beschreibt wasserlösliche, curcuminhaltige Nanopartikel zur Krebstherapie. Dabei soll der Einbau von Curcumin in Nanopartikel zu einer erhöhten Stabilität, Löslichkeit und Bioverfügbarkeit führen.

In WO 2009/061152 A2 ist eine Zusammensetzung zur Schmerzprävention und -behandlung umfassend Curcumin oder ein pharmazeutisch verträgliches Salz davon beschrieben.

Zhao *et al.* (2011) offenbart eine Micro-Emulgierung von Bisdemethoxycurcumin mit Hilfe von pseudoternären Phasendiagrammen, kombiniert mit einem orthogonalen Test in einem Drei-Phasen-System.

Wu *et al.* (2011) beschreibt ein selbst-micro-emulgierendes Drug Delivery System zur Erhöhung der Curcumin-Bioverfügbarkeit.

In Mosely *et al.* (2007) wird unter anderem die pharmakologische Wirksamkeit verschiedener Curcuminderivate, wie etwa Demethoxycurcumin, Bisdemethoxycurcumin und EF-24, beschrieben.

Der vorliegenden Erfindung liegt die Aufgabe zugrunde, eine pharmazeutische Formulierung der eingangs genannten Art zu schaffen, die Curcumin in einer hinreichend lagerstabilen Form zur Verfügung stellt und die eine einfache und sichere intravenöse Administration ermöglicht.

Gegenstand der Erfindung ist eine pharmazeutische Formulierung, die Curcumin und/oder ein Curcuminderivat gelöst in einem Alkohol sowie ferner eine Säure und einen Lösungsvermittler enthält, wobei das Curcuminderivat ausgewählt ist aus der Gruppe bestehend aus Demethoxycurcumin, Bisdemethoxycurcumin und EF-24. Die erfindungsgemäße Formulierung enthält höchstens 12 Gew.-% Wasser.

Zunächst seien einige im Rahmen der Erfindung verwendete Begriffe erläutert. Eine pharmazeutische Formulierung ist eine Zusammensetzung, die entweder unmittelbar als Endprodukt zu pharmazeutischen Zwecken eingesetzt werden kann oder aber als Zwischenprodukt (vorzugsweise lagerfähiges Zwischenprodukt) auf einfache Art und Weise in einer klinischen Umgebung vom medizinischen Personal in eine anwendungsfertige Form gebracht werden kann. Sie enthält somit lediglich pharmazeutisch akzeptable Bestandteile.

Der Begriff Curcuminderivat bezeichnet im Rahmen der Erfindung näturliche und synthetische Curcuminderivate. Beispielhaft genannt seien natürlich vorkommende Curcuminoide. Dies sind sekundäre Pflanzeninhaltsstoffe, die in den Rhizomen verschiedener Curcuma-Gewächse wie z.B. Curcuma longa vorkommen. Unter dem Begriff Curcuminoide werden die drei Substanzen Curcumin, Demethoxycurcumin und Bisdemethoxycurcumin zusammengefasst. Chemisch betrachtet handelt es sich bei den Curcuminoiden um konjugierte Diarylheptanoide, im weiteren Sinne also Polyphenole.

**Tabelle 1: Physikalische und chemische Eigenschaften von Curcuminoiden [Govindarajan, 1980; Pedersen et al., 1985; Tønnesen et al., 1995].**

| **Trivialname** | **CUR** | **Demethoxy-CUR** | **Bisdemethoxy-CUR** |
|---|---|---|---|
| Chemischer Name | D iferuloylmethan | 4-Hydroxycinnamoylferuloylmethan | Bis-4-Hydroxycinnamoylmethan |
| Summenformel | C₂₁H₂₀O₆ | C₂₀H₁₈O₅ | C₁₉H₁₆O₄ |
| Molekulargewicht g/mol) | 368,39 | 338,36 | 308,33 |
| Aussehen | gelbes, kristallines Pulver | orange-gelbes, amorphes Produkt | gelbe Platten |
| Schmelzpunkt (°C) | 182-183 | 172-174 | 223-224 |
| Äbsorptionsmax. (EtOH) | 427 | 424 | 418 |
| Löslichkeit | unlöslich in Wasser, Hexan, Ether; löslich in Alkohol, Aceton, Eisessig, organischen Lösungsmitteln | | |

Des Weiteren können synthetisch abgewandelte Moleküle hergestellt werden, die gleiche oder ähnliche physikalischchemische eigenschaften aufweisen, aber physiologisch wirksamer sein können. Ein Beispiel dafür ist EF-24:

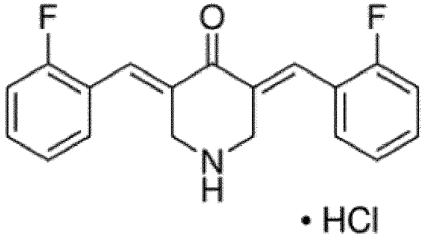

EF-24 ist ein IKK-Inhibitor und synthetisches Curcuminanalogon. EF-24 ist potenter als Curcumin und weist eine deutlich höhere Bioverfügbarkeit auf, außerdem besitzt es eine 10fach höhere Potenz in der Zelltodinduktion. Im Anti-Tumor-Screening zeigt es sich effektiver als Cisplatin mit wesentlich geringerem Nebenwirkungspotential.

Die Curcuminderivate sind somit erfindungsgemäß bevorzugt ausgewählt aus der Gruppe bestehend aus Demethoxycurcumin, Bis demethoxycurcumin und EF-24.

Curcumin liegt in verschiedenen tautomeren Formen vor, es existiert eine Keto-Enol-Tautomerie zwischen einer Keto-Form und zwei äquivalenten Enol-Formen. Die Keto-Enol-Struktur stellt die instabile Stelle des Curcumin-Moleküls dar.

Die chemische Stabilität von Curcumin in wässrigen Lösungen ist pH-abhängig. Unter neutralen bis basischen Bedingungen ist Curcumin in wässriger Lösung nicht stabil. In alkalischer Lösung erfolgt zuerst die Dissoziation des Enols (pKa 7, 8), wodurch die negative Ladung über den Aromaten stabilisiert und die konjugierte Dien-Struktur zerstört wird. Im Alkalischen folgt weiterhin die sukzessive Dissoziation der Phenole (pKa 8,5 und 9,0).

Da die Hydroxylgruppen bei saurem pH-Wert in undissoziierter Form vorliegen, weisen die Curcuminoide in diesem Milieu eine höhere Stabilität auf. Die Erfindung hat erkannt, dass Curcumin, wenn es in die Blutbahn gelangt, nicht durch den höheren physiologischen pH-Wert zerfällt, sondern an Plasma-Eiweiß gebunden wird und somit im Blutkreislauf verbleibt. Ein die Anmelderin nicht bindender und den Schutzbereich nicht beschränkender Erklärungsversuch ist, dass Curcumin eine lipophile und polyphenolische Verbindung darstellt, und somit zur Interaktion mit Makromolekülen imstande ist.

Die erfindungsgemäße Formulierung enthält bevorzugt höchstens 5 Gew.-%, weiter vorzugsweise höchstens 3 Gew.-% Wasser, weiter bevorzugt ist sie wasserfrei. Wasserfrei bedeutet, dass Wasser nicht oder allenfalls in solchen Mengen zugegen ist, dass das oben beschriebene Keto-Enol-Gleichgewicht nicht gestört wird.

Die Erfindung stellt Curcumin bzw. ein Curcuminderivat als lagerfähige, verhältnismäßig konzentrierte Lösung zur Verfügung, aus der erfindungsgemäßen Formulierung lässt sich auf einfache Art und Weise und in klinischer Umgebung eine Infusionslösung herstellen, in der das Curcumin in wässriger Umgebung gelöst und daher ohne weiteres intravenös administrierbar ist. Die Erfindung hat erkannt, dass sich Curcumin in eine verhältnismäßig konzentrierte alkoholische Lösung bringen lässt, in der es stabil lagerfähig ist, die Lagerfähigkeit eines solchen Konzentrats kann erfindungsgemäß 2 Jahre oder mehr betragen.

Die in dem alkoholischen Konzentrat vorhandene Säure führt dazu, dass sich bei der Herstellung einer Infusionslösung aus der erfindungsgemäßen Formulierung in dem dann wässrigen Milieu ein physiologisch akzeptabler pH-Wert einstellt. Die resultierende Infusionslösung weist bevorzugt einen pH-Wert im schwach sauren Milieu auf. Bspw. wird durch Zugabe der Zitronensäure im Konzentrat erreicht, dass sich in der wässrigen Infusionslösung ein pH-wert von 5,5 - 6,0 einstellt, der über die gesamte Infusionsdauer stabil ist. Der erfindungsgemäße Lösungsvermittler sorgt dafür, dass nach der Herstellung einer wässrigen Infusionslösung aus der pharmazeutischen Formulierung Curcumin in Lösung bleibt und in der wässrigen Umgebung nicht ausfällt.

Für den als Lösungsmittel verwendeten Alkohol ist Ethanol besonders bevorzugt. Der Alkoholgehalt beträgt bevorzugt 40 bis 90 Gew.-%, weiter vorzugsweise 40 bis 70 Gew.-%.

Als Säuren kommen prinzipiell anorganische Säuren, wie z.B. Phosphorsäure oder Salzsäure in Frage. Organische Säuren sind bevorzugt. Geeignete organische Säuren sollten bevorzugt in Reinform kristallin vorliegen, vorteilhafterweise zumindest aber in einer wasserfreien Form vorliegen. Sie sollten bevorzugt zu mindestens 1 Gew.-%% in dem verwendeten Alkohol löslich sein. Das Säureanion darf keine toxische Substanz sein, da es i.v. verträglich sein muss. Bevorzugte organische Säuren sind Weinsäure, Bernsteinsäure, Essigsäure, besonders bevorzugt Zitronensäure und Ascorbinsäure.

Ascorbinsäure hält den Zerfall von Curcumin in wässrigem Medium auf und stabilisiert somit auch eine aus dem erfindungsgemäßen Konzentrat hergestellte wässrige Infusionslösung. Ascorbinsäure ist physiologisch wirksam und wird ebenfalls therapeutisch als Infusionslösung eingesetzt.

Die Lösungsvermittler sind bevorzugt ausgewählt aus der Gruppe oberflächenaktiver Stoffe (Tenside bzw. Surfactants) und solubilisierenden Polymeren. Unter den solubilisierenen Polymeren sind besonders bevorzugt die Polyvinylpyrrolidone, Addukte von Ethylenoxid an Rizinusöl, Polyethylenglykole und Polysorbate. Geeignete Lösungsvermittler sind beispielsweise von der Firma BASF erhältlich und beschrieben in der Broschüre "Solubility Enhancement with BASF Pharma Polymers" (Solubilizer Compendium, Oktober 2011, Herausgeber Thomas Reintjes).

Ein geeignetes Addukt von Ethylenoxid an Rizinusöl ist beispielsweise von BASF unter der Bezeichnung Kolliphor ELP erhältlich.

Unter den Polysorbaten ist beispielsweise Polysorbat 80 (auch Tween 80 genannt) geeignet.

Die erfindungsgemäße pharmazeutische Formulierung kann bevorzugt 0,2 bis 3 Gew.-%, vorzugsweise 0,5 bis 2 Gew.-%, weiter vorzugsweise 0,5 bis 1,5 Gew.-% Curcumin oder Curcuminderivat enthalten. Der Gehalt an Zitronensäure (sofern enthalten) beträgt bevorzugt 0,2 bis 1 Gew.-%, weiter vorzugsweise 0,3 bis 0,5 Gew.-%. Der Gehalt an Ascorbinsäure (sofern enthalten) beträgt bevorzugt 0,05 bis 0,4 Gew.-%, vorzugsweise 0,07 bis 0,15 Gew.-%.

Das Verhältnis von Zitronensäure (sofern enthalten) zu Curcumin oder Curcuminderivat beträgt erfindungsgemäß bevorzugt 10 bis 100 Gewichtsteile, vorzugsweise 15 bis 50 Gewichtsteile Zitronensäure pro 100 Gewichtsteilen Curcumin oder Curcuminderivat Bei Zusatz von Ascorbinsäure werden bevorzugt 3 bis 30 Gewichtsteile, vorzugsweise 4 bis 20 Gewichtsteile Ascorbinsäure pro 100 Gewichtsteilen Curcumin oder Curcuminderivat eingesetzt.

Das Gewichtsverhältnis von Alkohol und Lösungsvermittler beträgt bevorzugt 2:1 bis 1:4, weiter vorzugsweise 1:1 bis 1:3.

Gegenstand der Erfindung ist ferner ein Verfahren zur Herstellung einer erfindungsgemäßen pharmazeutischen Formulierung. Das Verfahren weist folgende Schritte auf:
a. Lösen des Curcumin oder Curcuminderivats in Alkohol,
b. Zugabe der Säure,
c. Mischen des Solubilisators mit Alkohol,
d. Mischen der in b) und c) erhaltenen Komponenten.

Es wird zunächst eine Curcuminlösung in Alkohol (bevorzugt Ethanol) hergestellt, dabei kann es bevorzugt sein, zu erwärmen (beispielsweise auf etwa 70° C), um den Lösevorgang zu fördern. Die Säure kann bereits im Alkohol gelöst vorliegen oder in der warmen alkoholischen Curcuminlösung gelöst werden..

In einem weiteren Schritt wird eine einphasige Mischung des Solubilisators mit Alkohol hergestellt, diese in Schritt c) hergestellte Lösung wird zu der Curcuminlösung zugegeben, bis das gewünschte Volumen der pharmazeutischen Formulierung erreicht ist.

Gegenstand der Erfindung ist ferner eine Infusionslösung, die eine Trägerlösung und eine darin gelöste pharmazeutische Formulierung gemäß der vorliegenden Erfindung enthält. Bei der Trägerlösung kann es sich erfindungsgemäß insbesondere um isotonische Kochsalzlösung, eine Glucoselösung (beispielsweise 5%ige wässrige Glucoselösung) oder eine sonstige für Infusionszwecke akzeptable Lösung handeln.

Bei der erfindungsgemäßen Infusionslösung handelt es sich um eine einphasige Lösung, in der Curcumin bzw. das Curcuminderivat gelöst vorliegt. Die in der erfindungsgemäßen pharmazeutischen Formulierung enthaltenen Lösungsvermittler bewirken, dass beim Herstellen der erfindungsgemäßen Infusionslösung aus der erfindungsgemäßen pharmazeutischen Formulierung und der Trägerlösung das Curcumin bzw. Curcuminderivat nicht ausfällt, sondern in Lösung bleibt. Die in der pharmazeutischen Formulierung enthaltene Säure stellt im wässrigen Milieu der Infusionslösung den gewünschten physiologischen pH-Wert ein.

Bei Verwendung einer isotonischen Kochsalzlösung als Trägerlösung entsteht in der erfindungsgemäßen Infusionslösung ein Zitronensäure/Citratpuffer, der den gewünschten pH-Wert (bevorzugt 5 bis 7, weiter vorzugsweise 5,5 bis 6,5) einstellt und puffert.

Eine erfindungsgemäße Infusionslösung kann bevorzugt 0,2 bis 4 mg, weiter vorzugsweise 0,5 bis 1 mg Curcumin oder Curcuminderivat pro ml Infusionslösung enthalten. Beispielsweise kann im Rahmen der Erfindung 500 ml Infusionslösung zwischen 250 und 450 mg Curcumin enthalten. Durch intravenöse Gabe der Infusionslösung über einen Zeitraum von beispielsweise 90 min lässt sich eine für eine systemische Therapie erforderliche Curcuminmenge administrieren.

Gegenstand der Erfindung ist ferner eine erfindungsgemäße pharmazeutische Formulierung oder Infusionslösung zur Verwendung als Medikament, insbesondere antineoplastisches Medikament.

Ausführungsbeispiele der Erfindung werden nachfolgend beschrieben.

### Beispiel 1

100 mg Curcumin werden in 3,8 ml absolutem Ethanol unter Rühren und Erwärmen auf etwa 70° C gelöst. Nach 15 min hat sich eine klare, dunkelgelbe Lösung gebildet.

In dieser Lösung werden 40,7 mg Zitronensäure wasserfrei gelöst.

25,88 g Kalliphor ELP werden mit 12,37 g absolutem Ethanol gemischt. Mit der so hergestellten Mischung wird die vorstehend hergestellte Curcuminlösung auf 10 ml aufgefüllt.

Die erhaltene Lösung wird steril filtriert und bei 121° C autoklaviert.

Man erhält so eine lagerfähige pharmazeutische Formulierung, die Curcumin in gelöster Form enthält.

### Beispiel 2

Zur Herstellung einer Infusionslösung wird die pharmazeutische Formulierung gemäß Beispiel 1 in eine Trägerlösung (bevorzugt isotonische Kochsalzlösung oder 5%ige Glucoselösung) gegeben, sodass sich in dieser Infusionslösung bevorzugt eine Konzentration des Curcumins von 0,5 bis 1 mg/ml einstellt.

### Beispiel 3

Bei diesem Beispiel wird als Lösungsmittel ein Alkohol mit Wasseranteil verwendet.

100 mg Curcumin werden in 3,8 ml 70%igem Ethanol (Rest Wasser) unter Rühren und Erwärmen auf etwa 70° C gelöst. Nach 15 min hat sich eine klare, dunkelgelbe Lösung gebildet.

In dieser Lösung werden 10 mg Ascorbinsäure wasserfrei gelöst.

25,88 g Kalliphor ELP werden mit 12,37 g absolutem Ethanol gemischt. Mit der so hergestellten Mischung wird die vorstehend hergestellte Curcuminlösung auf 10 ml aufgefüllt.

Die erhaltene Lösung wird steril filtriert und bei 121° C autoklaviert.

Man erhält so eine lagerfähige pharmazeutische Formulierung, die Curcumin in gelöster Form enthält. Der Wasseranteil beträgt etwa 12 Gew.-%.

In den nachfolgenden Besipielen 4 und 5 wird eine erhöhte Konzentration von Curcumin in der pharmazeutischen Formulierung in Lösung gebracht.

### Beispiel 4

150 mg Curcumin werden in 4 ml absolutem Ethanol unter Rühren und Erwärmen auf etwa 70° C gelöst. Nach 15 min hat sich eine klare, dunkelgelbe Lösung gebildet.

In dieser Lösung werden 34 mg Zitronensäure wasserfrei gelöst.

25,88 g Kalliphor ELP werden mit 12,37 g absolutem Ethanol gemischt. Mit der so hergestellten Mischung wird die vorstehend hergestellte Curcuminlösung auf 10 ml aufgefüllt.

Die erhaltene Lösung wird steril filtriert und bei 121° C autoklaviert.

Man erhält so eine lagerfähige pharmazeutische Formulierung, die Curcumin in gelöster Form enthält.

### Beispiel 5

150 mg Curcumin werden in 4 ml absolutem Ethanol unter Rühren und Erwärmen auf etwa 70° C gelöst. Nach 15 min hat sich eine klare, dunkelgelbe Lösung gebildet.

In dieser Lösung werden 10 mg Ascorbinsäure wasserfrei gelöst.

25,88 g Kalliphor ELP werden mit 12,37 g absolutem Ethanol gemischt. Mit der so hergestellten Mischung wird die vorstehend hergestellte Curcuminlösung auf 10 ml aufgefüllt.

Die erhaltene Lösung wird steril filtriert und bei 121° C autoklaviert.

Man erhält so eine lagerfähige pharmazeutische Formulierung, die Curcumin in gelöster Form enthält.

## Patentansprüche

1. Pharmazeutische Formulierung, **dadurch gekennzeichnet, dass** sie Curcumin und/oder ein Curcuminderivat, einen Alkohol, eine Säure, sowie einen Lösungsvermittler enthält und höchstens 12 Gew.-% Wasser enthält,
wobei das Curcuminderivat ausgewählt ist aus der Gruppe bestehend aus Demethoxycurcumin, Bisdemethoxycurcumin und EF-24.

2. Pharmazeutische Formulierung nach Anspruch 1, **dadurch gekennzeichnet, dass** der Alkohol Ethanol ist und/oder wobei der Alkoholgehalt 40 bis 90 Gew.-% vorzugsweise 40 bis 70 Gew.-% betragen kann.

3. Pharmazeutische Formulierung nach Anspruch 1 oder 2, **dadurch gekennzeichnet, dass** sie höchstens 5 Gew.-% Wasser enthält, weiter vorzugsweise höchstens 3 Gew.-% Wasser weiter vorzugsweise wasserfrei ist.

4. Pharmazeutische Formulierung nach einem der Ansprüche 1 bis 3, **dadurch gekennzeichnet, dass** die Säure Ascorbinsäure oder eine Lebensmittelsäure, vorzugsweise Zitronensäure ist.

5. Pharmazeutische Formulierung nach einem der Ansprüche 1 bis 4, **dadurch gekennzeichnet, dass** die Lösungsvermittler ausgewählt sind aus der Gruppe bestehend aus oberflächenaktiven Stoffen (surfactants) und solubilisierenden Polymeren wobei die solubilisierenden Polymere ausgewählt sein können aus der Gruppe bestehend aus Polyvinylpyrrolidonen, Addukten von Ethylenoxid an Rizinusöl, und Polysorbaten.

6. Pharmazeutische Formulierung nach einem der Ansprüche 1 bis 5, **dadurch gekennzeichnet, dass** sie 0,2 bis 3 Gew.-% vorzugsweise 0,5 bis 2 Gew.-% weiter vorzugsweise 0,5 bis 1,5 Gew.-% Curcumin oder Curcuminderivat enthält.

7. Pharmazeutische Formulierung nach einem der Ansprüche 1 bis 6, **dadurch gekennzeichnet, dass** sie 0,2 bis 1 Gew.-% vorzugsweise 0,3 bis 0,5 Gew.-% Zitronensäure enthält oder dass sie 0,05 bis 0,4 Gew.-% vorzugsweise 0,07 bis 0,15 Gew.-% Ascorbinsäure enthält.

8. Pharmazeutische Formulierung nach einem der Ansprüche 1 bis 7, **dadurch gekennzeichnet, dass** sie sie 10 bis 100 Gewichtsteile, vorzugsweise 15 bis 50 Gewichtsteile Zitronensäure pro 100 Gewichtsteilen Curcumin oder Curcuminderivat enthält oder dass sie sie 3 bis 30 Gewichtsteile, vorzugsweise 4 bis 20 Gewichtsteile Ascorbinsäure pro 100 Gewichtsteilen Curcumin oder Curcuminderivat enthält.

9. Pharmazeutische Formulierung nach einem der Ansprüche 1 bis 8, **dadurch gekennzeichnet, dass** das Gewichtsverhältnis von Alkohol und Lösungsvermittler 2:1 bis 1:4, vorzugsweise 1:1 bis 1:3 beträgt.

10. Verfahren zur Herstellung einer pharmazeutischen Formulierung nach einem der Ansprüche 1 bis 9, **gekennzeichnet durch** folgende Schritte:
a) Lösen des Curcumin oder Curcuminderivats in Alkohol,
b) Zugabe der Säure,
c) Mischen des Solubilisators mit Alkohol,
d) Mischen der in b) und c) erhaltenen Komponenten.

11. Infusionslösung, **dadurch gekennzeichnet, dass** sie eine Trägerlösung und eine darin gelöste pharmazeutische Formulierung nach einem der Ansprüche 1 bis 9 enthält, wobei die Trägerlösung ausgewählt sein kann aus der Gruppe isotonische Kochsalzlösung und Glukoselösung.

12. Infusionslösung nach Anspruch 11, **dadurch gekennzeichnet, dass** sie 0,2 bis 4 mg/ml, bevorzugt 0,5 bis 1 mg/ml Curcumin oder Curcuminderivat enthält.

13. Pharmazeutische Formulierung nach einem der Ansprüche 1 bis 9 zur Verwendung als Medikament.

14. Infusionslösung nach Anspruch 11 oder 12, zur Verwendung als Medikament.

## Claims

1. Pharmaceutical formulation, **characterised in that** it contains curcumin and/or a curcumin derivative, an alcohol, an acid, and a solubiliser and a maximum of 12% by weight of water, wherein the curcumin derivative is selected from the group comprising demethoxycurcumin, bisdemethoxycurcumin and EF-24.

2. Pharmaceutical formulation according to claim 1, **characterised in that** the alcohol is ethanol and/or wherein the alcohol content may be from 40 to 90% by weight, preferably from 40 to 70% by weight.

3. Pharmaceutical formulation according to claim 1 or claim 2, **characterised in that** it contains a maximum of 5% by weight of water, more preferably a maximum of 3% by weight of water, more preferably is water-free.

4. Pharmaceutical formulation according to any one of claims 1 to 3, **characterised in that** the acid is ascorbic acid or a food acid, preferably citric acid.

5. Pharmaceutical formulation according to any one of claims 1 to 4, **characterised in that** the solubilisers are selected from the group comprising surfactants and solubilising polymers, wherein the solubilising polymers may be selected from the group comprising polyvinylpyrrolidones, adducts of ethylene oxide to castor oil, and polysorbates.

6. Pharmaceutical formulation according to any one of claims 1 to 5, **characterised in that** it contains from 0.2 to 3% by weight, preferably from 0.5 to 2% by weight, more preferably from 0.5 to 1.5% by weight of curcumin or curcumin derivative.

7. Pharmaceutical formulation according to any one of claims 1 to 6, **characterised in that** it contains from 0.2 to 1% by weight, preferably from 0.3 to 0.5% by weight of citric acid, or **in that** it contains from 0.05 to 0.4% by weight, preferably from 0.07 to 0.15% by weight of ascorbic acid.

8. Pharmaceutical formulation according to any one of claims 1 to 7, **characterised in that** it contains from 10 to 100 parts by weight, preferably from 15 to 50 parts by weight of citric acid per 100 parts by weight of curcumin or curcumin derivative or **in that** it contains from 3 to 30 parts by weight, preferably from 4 to 20 parts by weight of ascorbic acid per 100 parts by weight of curcumin or curcumin derivative.

9. Pharmaceutical formulation according to any one of claims 1 to 8, **characterised in that** the weight ratio of alcohol and solubiliser is from 2:1 to 1:4, preferably from 1:1 to 1:3.

10. Method for producing a pharmaceutical formulation according to any one of claims 1 to 9, **characterised by** the following steps:
a) dissolving the curcumin or curcumin derivative in alcohol,
b) adding the acid,
c) mixing the solubiliser with alcohol,
d) mixing the components contained in b) and c).

11. Infusion solution, **characterised in that** it contains a carrier solution and a pharmaceutical formulation dissolved therein according to any one of claims 1 to 9, wherein the carrier solution may be selected from the group isotonic saline solution and glucose solution.

12. Infusion solution according to claim 11, **characterised in that** it contains from 0.2 to 4 mg/ml, preferably from 0.5 to 1 mg/ml of curcumin or curcumin derivative.

13. Pharmaceutical formulation according to any one of claims 1 to 9 for use as a medication.

14. Infusion solution according to claim 11 or 12 for use as a medication.

## Revendications

1. Formulation pharmaceutique, **caractérisée en ce qu'**elle contient de la curcumine et/ou un dérivé de curcumine, un alcool, un acide, ainsi qu'un tiers-solvant, et contient au plus 12 % en poids d'eau, le dérivé de curcumine étant choisi dans le groupe consistant en la déméthoxycurcumine, la bisdéméthoxycurcumine et l'EF-24.

2. Formulation pharmaceutique selon la revendication 1, **caractérisée en ce que** l'alcool est l'éthanol, et/ou la teneur en alcool pouvant être de 40 à 90 % en poids, de préférence de 40 à 70 % en poids.

3. Formulation pharmaceutique selon la revendication 1 ou 2, **caractérisée en ce qu'**elle contient au plus 5 % en poids d'eau, plus préférentiellement au plus 3 % en poids d'eau, et d'une manière encore plus préférée est anhydre.

4. Formulation pharmaceutique selon l'une des revendications 1 à 3, **caractérisée en ce que** l'acide est l'acide ascorbique ou un acide alimentaire, de préférence l'acide citrique.

5. Formulation pharmaceutique selon l'une des revendications 1 à 4, **caractérisée en ce que** le tiers-solvant est choisi dans le groupe consistant en les substances tensioactives (surfactants) et les polymères solubilisants, les polymères solubilisants pouvant être choisis dans le groupe consistant en les polyvinylpyrrolidones, les produits d'addition oxyéthylénés de l'huile de ricin, et les polysorbates.

6. Formulation pharmaceutique selon l'une des revendications 1 à 5, **caractérisée en ce qu'**elle contient 0,2 à 3 % en poids, de préférence 0,5 à 2 % en poids, plus préférentiellement 0,5 à 1,5 % en poids de curcumine ou du dérivé de curcumine.

7. Formulation pharmaceutique selon l'une des revendications 1 à 6, **caractérisée en ce qu'**elle contient 0,2 à 1 % en poids, de préférence 0,3 à 0,5 % en poids d'acide citrique, ou qu'elle contient 0,05 à 0,4 % en poids, de préférence 0,07 à 0,15 % en poids d'acide ascorbique.

8. Formulation pharmaceutique selon l'une des revendications 1 à 7, **caractérisée en ce qu'**elle contient 10 à 100 parties en poids, de préférence 15 à 50 parties en poids d'acide citrique pour 100 parties en poids de curcumine ou du dérivé de curcumine, ou qu'elle contient 3 à 30 parties en poids, de préférence 4 à 20 parties en poids d'acide ascorbique pour 100 parties en poids de curcumine ou du dérivé de curcumine.

9. Formulation pharmaceutique selon l'une des revendications 1 à 8, **caractérisée en ce que** le rapport en poids de l'alcool au tiers-solvant est de 2:1 à 1:4, de préférence de 1:1 à 1:3.

10. Procédé de fabrication d'une formulation pharmaceutique selon l'une des revendications 1 à 9, **caractérisé par** les étapes suivantes :
a) dissolution de la curcumine ou du dérivé de curcumine dans un alcool,
b) addition de l'acide,
c) mélange du solubilisant à l'alcool,
d) mélange des composants obtenus en b) et c).

11. Solution pour perfusion, **caractérisée en ce qu'**elle contient une solution support et une formulation pharmaceutique, qui y est dissoute, selon l'une des revendications 1 à 9, la solution support pouvant être choisie dans le groupe consistant en une solution physiologique salée isotonique et une solution de glucose.

12. Solution pour perfusion selon la revendication 11, **caractérisée en ce qu'**elle contient 0,2 à 4 mg/ml, de préférence 0,5 à 1 mg/ml de curcumine ou du dérivé de curcumine.

13. Formulation pharmaceutique selon l'une des revendications 1 à 9 pour une utilisation en tant que médicament.

14. Solution pour perfusion selon la revendication 11 ou 12, pour une utilisation en tant que médicament.
